Europäisches Patentamt

European Patent Office

Office européen des brevets

(11) Publication number: **0 212 703 B1**

# EUROPEAN PATENT SPECIFICATION

(45) Date of publication of patent specification: **04.09.91**  (51) Int. Cl.⁵: **A61K  39/215**, A61K 39/17

(21) Application number: **86201224.2**

(22) Date of filing: **14.07.86**

(54) Infectious bronchitis vaccines for day-old chickens.

(30) Priority: **18.07.85 NL 8502070**

(43) Date of publication of application:
**04.03.87 Bulletin  87/10**

(45) Publication of the grant of the patent:
**04.09.91 Bulletin  91/36**

(84) Designated Contracting States:
**AT BE CH DE FR GB IT LI LU NL SE**

(56) References cited:
**EP-A- 0 030 063**
**EP-A- 0 073 547**
**EP-A- 0 086 025**

**BIOLOGICAL ABSTRACTS, volume 62, 15.7.1976, abstract 8254 Philadelphia, Pa (US), R.W. Winterfield et al. " Immunity from spray vaccination with derivatives of a Holland strain" & Avian Dis 20 (1), 42-48, 1976**

**BIOLOGICAL ABSTRACTS, volume 72, 1981, abstract 24308 Philadelphia, Pa (US), P.J. Wyeth et al "Immune response of breeding chickens to trivalent on emulsion vaccines. Responses to Newcastle disease and infectious bursa disease" & Vet.Rec. 198(4), 72-75, 1981**

(73) Proprietor: **DUPHAR INTERNATIONAL RE-
SEARCH B.V**
**C.J. van Houtenlaan 36**
**NL-1381 CP Weesp(NL)**

(72) Inventor: **van Wiltenburg, Nico**
**OCTROOIBUREAU ZOAN B.V. P.O. Box 140**
**NL-1380 AC Weesp(NL)**

(74) Representative: **Muis, Maarten et al**
**OCTROOIBUREAU ZOAN B.V. P.O. Box 140**
**NL-1380 AC Weesp(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The invention relates to infectious bronchitis virus (IBV) vaccines for poultry and more in particular to improved infectious bronchitis vaccines which are extremely safe and effective for vaccinating day-old chickens.

The use of live infectious bronchitis vaccines for poultry has been known for a great many years already. Infectious bronchitis is an important affection of the bronchial tubes in poultry caused by a corona virus. The affection causes great economic losses in poultry keeping, since infectious bronchitis still causes enormous mortality especially in young poultry. Besides mortality and more or less strong respiratory phenomena, damage to the hen's egg laying system and hence reduction in production occurs in laying hens as a result of an IB infection. Moreover, infections with IBV may stimulate virus and bacterial infections and thus result in serious economic losses, in particular in the broiler sector.

Vaccines on the basis of live virus have initially been used to control infectious bronchitis. For example, live IBV vaccines based on a modified H-strain have been used for a long time already. The original H-strain was isolated by Bijlenga, Hoekstra and Rispens (Tijdschrift voor Diergeneeskunde, 81, (1956), p. 43, and 85 (1960), p. 279, and 85 (1960), p. 398) and was passed a few times over hatched hen's eggs. Eventually, strains (H120 and H52) were obtained which proved to be less virulent for the hen than the virus isolated from the field. The modified strain H120 is used for vaccinating day-old chickens, while strain H52 is used especially in animals at an age of approximately 8 weeks.

Although the use of vaccines based on the strains H120 and H52 is fairly safe and effective, it has been found that the two live vaccines in certain circumstances are not capable of preventing outbreaks of infectious bronchitis. These outbreaks may be ascribed to the occurrence of modified strains. The occurrence of modified strains called for the search for more effective vaccines based on infectious bronchitis virus strains of different serotypes.

This investigation has resulted in the vaccines which are described in European Patent Specification 0,030,063 and European Patent Application 0,086,025 and which are both based on infectious bronchitis virus (IBV) modified strains. It has been found that an effective protection against infectious bronchitis is obtained by using vaccines (live and inactivated ones) based on the so-called modified strains.

Investigations have demonstrated that laying hens can be effectively protected by a combined use of both live and inactivated vaccines.

Broilers have so far been vaccinated against infectious bronchitis on the first day of life with a vaccine based on strain H120. However, due to the occurence of the modified strains, said vaccination is no longer effective and hence derivatives of the occurring modified strains ought to be found which can be administered on day 1. Investigation in broilers carried out by the Public Health Department for Poultry in Doorn has proved that besides the serotype Massachusetts (H120, H52), mainly viruses of the serotype D207 are found. Consequently, an effective vaccine for the protection of broilers against infectious bronchitis will have to consist of H120 and a virus related to strain D207.

Although it is stated in the above-mentioned European Patent Specification and the European Patent Application that the vaccines described therein can also be used in young animals, experiments have proved that live vaccines based on the IBV-modified strains known from these documents are too virulent for the vaccination of day-old broilers. The strain D274 mentioned in the European Patent Application (and registered in Central Veterinary Laboratory, New Haw, Weybridge, England VLO 10110/AVI/3; and Institut Pasteur No. I-216, respectively) proved to be too virulent for vaccination of day-old chickens after 52 and even after 122 egg passages.

It has surprisingly been found that a safe and effective live vaccine for the protection of day-old chickens against a modified strain of the infectious bronchitis is obtained when the strain registered in the Institut Pasteur under No. I-473 is used as a virus strain.

The virus strain used is derived from a strain isolated from the field and will hereinafter be referred to as virus strain 84-3.

A generally known problem in administering vaccines based on more than one live virus strain is the fact that the combined viruses become less immunogenic due to a mutual interaction (see Am.J.Vet.Res. 36, (1965), p. 4, 524 and 525; and Avian Diseases 12, (1968), p. 577).

It is extraordinarily surprising that the problem of interference does not present itself when the new strain 84-3 according to the invention is combined with H120 and other live poultry viruses.

Experiment 1: Safety of the strain 84-3.

Three groups of 20 day-old SPF chickens each were vaccinated on day 1 with a dose of $10^{4.3}$ EID$_{50}$ -

(egg infective dose) per chicken by means of dripping in the eye (group 1 with the known strain H120; group 2 with the strain D274 after it had been subjected to 52 egg passages; group 3 with the strain 84-3 according to the invention). A fourth group of 20 animals served as the control group.

On day 5, 10 chickens of each group were killed for tissue investigation of the trachea (see R.W. Winterfield et al, Avian Dis. 16 (1972), pp. 260-269). The remaining 10 chickens of each group were also killed on day 5 for investigation of ciliary activity (J.H. Darbyshire, Avian Pathol. 9 (1980), pp. 179-184). The results of this experiment are recorded in Table A:

TABLE A

| Group | Ciliary activity [a] | Trachea lesions [b] |
|---|---|---|
| 1 | 6/9 | 0.1 |
| 2 | 4/10 | 1.3 |
| 3 | 9/10 | 0.54 |
| control | 10/10 | 0 |

a) recorded is the number of chickens with ciliary activity and the total number of examined chickens per group.
b) the scale used in this test for the evaluation is from 0 to 3.

It appears from the above that, starting from a dose of $10^{4.3}$ $EID_{50}$ per animal, i.e. more than a 10-fold excess, based on ciliary activity the strain 84-3 is less virulent than the strain D274 after 52 eggs passages and the strain H120. However, based on trachea lesions, the strain 84-3 is slightly more virulent than the strain H120. An explanation for this difference could be that the strain 84-3 causes more lesions without destroying the ciliary activity, which then would mean that the strains 84-3 and H120 have slightly different pathology.

In general, the presence of ciliary activity is considered as a reliable indication for protection against a challenge with virulent virus.

Since 9 out of 10 chickens vaccinated with strain 84-3 showed ciliary activity, it could be concluded that the strain is safe for day-old chickens, the more so when it is considered that in this experiment more than a 10-fold excess has been administered to extremely sensitive animals, namely SPF-chickens without maternal immunity.

Experiment 2: Efficacy of the vaccination with strain 84-3.

Two groups of 30 day-old chickens each were housed separately.

On day 1, the test group was vaccinated by eye drop with a dose of $10^{3.3}$ $EID_{50}$ per animal (of the strain 84-3 according to the invention). Two weeks after vaccination, 5 chickens from each group were killed and examined for ciliary activity. This was repeated 4 weeks after vaccination. Both groups demonstrated 100% ciliary activity.

Four weeks after vaccination, 12 animals from each group were challenged with $10^{3.0-3.5}$ $EID_{50}$ per chicken with the virulent strain D207. The challenge virus was administered partly intranasally and partly intraocularly. Six days after the animals were examined for ciliary activity and trachea lesions. The results are recorded in Table B hereinafter:

TABLE B

| Group | Trachea lesions | Ciliary activity [c] | | | n |
|---|---|---|---|---|---|
| | | + | +/- | - | |
| test | 0.5 | 10 | 1 | 1 | 12 |
| control | 1.9 | 2 | 0 | 10 | 12 |

c) + : ciliary activity present
+/-: ciliary activity present at some places
- : no ciliary activity present.

3

As is demonstrated by the results obtained, more than 83% of the animals from the test group are protected, where as only 17% of the non-vaccinated control group are protected. This result is confirmed by the score of the trachea lesion examination in both groups.

Experiment 3: Combined administration of strain 84-3 and strain H120 to day-old chickens.

Two groups of 17 day-old SPF chickens each were vaccinated on day 1 with a dose of $10^{3.0}$ $EID_{50}$ of strain 84-3 + $10^{3.5}$ $EID_{50}$ of strain H120 per chicken. Each test group was accompanied by a control group of 8 chickens. Four weeks after the vaccination the animals of one test group and one control group were challenged with $10^{3.5}$ $EID_{50}$ of the virulent strain D207. The other test group and control group were challenged at the same point of time with $10^{3.5}$ $EID_{50}$ of the strain Voet (i.e. the virulent strain). Both virulent strains were administered both intranasally and intraocularly. Before administration of the challenge virus the vaccinated and non-vaccinated chickens were joined together. Six days after administration of the challenge virus the animals were killed and examined for ciliary activity and trachea lesions. The results are recorded in Table C:

TABLE C

| Group | Chall. with D207 | | Chall. with Voet | |
|---|---|---|---|---|
| | Ciliary act. | Trach. les. | Ciliary act. | Trach. les. |
| test | 17/17 | 0.51 | 17/17 | 0 |
| contr. | 0/8 | 1.63 | 0/8 | 2.63 |

From this it appears that the ciliary activity in the chickens of both groups was intact, which indicates 100% protection. This is confirmed by the low lesion score in the two test groups (0.51 and 0) with respect to the control groups (1.63 and 2.63).

Vaccination of day-old SPF chickens with a combination of the strains 84-3 and H120 provides a good protection against a challenge with virus of the strain D207 or the strain Voet, which indicates that both viruses do not adversely influence each other during the vaccination. This may once more appear from experiment 4:

Experiment 4: Field experiment on a small scale.

Three groups of 375 (group 1), 400 (group 2) and 50 (group 3) day-old broilers, and one group of 40 day-old SPF-chickens (group 4) were used.

Group 1 was vaccinated on day 0 with a dose of $10^{4.5}$ $EID_{50}$ of strain 84-3 and $10^{3.3}$ $EID_{50}$ of strain H120 per animal. On day 0, a dose of $10^{3.3}$ $EID_{50}$ of strain H120 per animal was administered to group 2 (spray method). Groups 3 and 4 served as non-vaccinated control groups.

On day 10, 20 animals of the groups 1 and 2 were killed for trachea lesion examination.

The breathing of the animals was checked from day 0 to day 21.

On day 28, 25 animals of the groups 1, 2 and 3 and 10 animals of group 4 were challenged intraocularly with a dose of $10^{3.8}$ $EID_{50}$ of the virulent strain Voet.

The same number of animals of each group were challenged on day 28 with a dose of $10^{3.5}$ $EID_{50}$ of the virulent strain D274 (intranasally and intraocularly).

Six days after administration of the challenge virus, the animals were killed and tested for trachea lesions and ciliary activity.

The challenge of day 28 was repeated with identical groups on day 42. These animals were also killed 6 days later and examined.

The remaining animals of groups 1 and 2 were weighed on day 60 so as to determine the average weight on the day of slaughter.

The results of the trachea lesion test after 10, 28 + 6 and 42 + 6 days are recorded in table D hereinafter:

4

TABLE D

| (Trachea lesion score) | | | | | |
|---|---|---|---|---|---|
| Group No. | 10 days after vaccination | 28 + 6 days after challenge with | | 42 + 6 days after challenge with | |
| | | Voet | D274 | Voet | D274 |
| 1 | 0.40 | 0.17 | 0.31 | 0.00 | 0.04 |
| 2 | 0.35 | 0.24 | 0.60 | 0.08 | 0.57 |
| 3 | n.d. | 1.59 | 1.83 | n.d. | n.d. |
| 4 | n.d. | 1.10 | 1.33 | 1.09 | 1.60 |
| n.d. = not done | | | | | |

The results of the cilia stopping test (CST) are recorded in Table E, in which the numbers indicate the percentage of protection.

TABLE E

| (CST) | | | | |
|---|---|---|---|---|
| Group | 28 + 6 days after challenge with | | 42 + 6 days after challenge with | |
| | Voet | D274 | Voet | D274 |
| 1 | 100 | 92 | 100 | 100 |
| 2 | 91 | 64 | 100 | 84 |
| 3 | 0 | 0 | n.d. | n.d. |
| 4 | 0 | 0 | 0 | 0 |

On the day of slaughter, the animals of group 1 had an average weight of 2350 g (n = 221), those of group 2 had an average weight of 2310 g (n = 233).

From the above results it follows that the combination of strain 84-3 according to the invention and H120 is safe for day-old broilers, because

1) no increase of respiratory symptoms occurs after administration of the combination vaccine;

2) there is no higher trachea lesion score after vaccination;

3) there is no significant difference in slaughter weight.

The protection after challenge with virulent virus of the strain D274 is much better for group 1 (92 and 100% after 4 and 6 weeks, respectively) than for group 2 (64 and 83%). Non-vaccinated animals are already fully affected at an age of 4 weeks.

These results were confirmed entirely by the trachea lesion score. Group 1: 0.31 and 0.04. Group 2: 0.60 and 0.57 at an age of 4 and 6 weeks, respectively. Group 3: 1.83 at an age of 4 weeks.

The protection against challenge with the strain Voet which is homologous for H120 is comparable in the groups 1 and 2.

Group 1: 100% after 4 and 6 weeks;

Group 2: 91% (4 weeks), 100% (6 weeks).

This is also confirmed by the trachea lesion score:

Group 1: 0.17 and 0.00 after 4 and 6 weeks, respectively;

Group 2: 0.24 and 0.08 after 4 and 6 weeks, respectively;

Group 3: 1.59 after 4 weeks.

So the combined vaccine with strain 84-3 and strain H120 gives an excellent protection against infection with both the strain D274 and against the Massachusetts strain during the whole fattening period.

On the basis of the above it will be clear that the present invention relates to new live IBV vaccines which are derived at least from the new IBV strain 84-3 and to the use thereof for vaccinating day-old chickens.

Vaccines are preferably derived from the H120 virus strain and from IBV of the strain 84-3. The new virus strain 84-3 can be processed to a live vaccine according to standard methods. In these methods, the virus is mixed with stabilisers conventionally used for this purpose and then freeze-dried. For administration purposes, the freeze-dried vaccine should be dissolved in a liquid solvent suitable for that purpose. The vaccine may be administered by means of the so-called eye drop, nose drop, drinking-water or spray methods for live vaccines. Vaccination by means of the new live vaccines according to the invention is preferably carried out in day-old poultry.

It will be obvious that combined live vaccines derived from the new IBV strain 84-3, strain H120 and from one or more other poultry virus types, for example, Newcastle disease virus, also form part of the present invention.

## Claims

1. A live vaccine against infectious bronchitis, characterized in that it comprises a virus of the strain IBV 84-3 which is registered in the Institut Pasteur in Paris under No. I-473.

2. A vaccine as claimed in Claim 1, characterized in that it comprises a quantity of virus of the strain 84-3 of at least $10^{2.5}$ EID$_{50}$ per dose.

3. A vaccine as claimed in Claim 1 or 2, characterized in that it also comprises one or more viruses of other IBV-strains.

4. A vaccine as claimed in Claim 3, characterized in that, besides virus of the strain 84-3, it also comprises virus of the strain H120.

5. A vaccine as claimed in Claim 4, characterized in that the overall quantity of IBV is at least $10^{6.0}$ EID$_{50}$ per dose.

6. A vaccine as claimed in Claims 1-5, characterized in that it also comprises one or more other poultry viruses.

7. A vaccine as claimed in Claim 6, characterized in that, besides virus of one or more IBV strains, it also comprises Newcastle Disease Virus.

8. The use of virus strain IBV 84-3, registered in the Institut Pasteur in Paris under No. I-473, for the preparation of a live vaccine suitable for vaccinating day-old chickens against infectious bronchitis.

9. A virus strain IBV 84-3 registered in the Institut Pasteur in Paris under No. I-473.

## Revendications

1. Vaccin vivant contre la bronchite infectieuse, caractérisé en ce qu'il comprend un virus appartenant à la souche VBI 84-3 qui est déposée à l'Institut Pasteur à Paris sous le n° I-473.

2. Vaccin selon la revendication 1, caractérisé en ce qu'il comprend une quantité d'au moins $10^{2,5}$ DIE$_{50}$ - (dose infectieuse pour oeuf) du virus de la souche 84-3 par dose.

3. Vaccin selon la revendication 1 ou 2, caractérisé en ce qu'il comprend également un ou plusieurs virus appartenant à d'autres souches de VBI.

4. Vaccin selon la revendication 3, caractérisé en ce que, outre le virus appartenant à la souche 84-3, il comprend également un virus appartenant à la souche H120.

5. Vaccin selon la revendication 4, caractérisé en ce que la quantité totale de VBI est d'au moins $10^{6,0}$ DIE$_{50}$ par dose.

6. Vaccin selon les revendications 1 à 5, caractérisé en ce qu'il comprend également un ou plusieurs autres virus de la volaille.

7. Vaccin selon la revendication 6, caractérisé en ce que, outre un ou des virus appartenant à une ou plusieurs souches de VBI, il comprend également le virus de la maladie de Newcastle.

8. Utilisation de la souche virale VBI 84-3, déposée à l'Institut Pasteur à Paris sous le n° I-473, pour la préparation d'un vaccin vivant approprié à la vaccination de poussins d'un jour contre la bronchite infectieuse.

9. Souche virale VBI 84-3 déposée à l'Institut Pasteur à Paris sous le n° I-473.

**Patentansprüche**

1. Lebendimpfstoff gegen infektiöse Bronchitis, dadurch gekennzeichnet, daß er ein Virus des Stammes IBV 84-3, der beim Institut Pasteur in Paris unter der Nr. I-473 eingetragen ist, umfaßt.

2. Impfstoff nach Anspruch 1, dadurch gekennzeichnet, daß er eine Menge an Virus des Stammes 84-3 von wenigstens $10^{2.5}$ EID$_{50}$ pro Dosis umfaßt.

3. Impfstoff nach Anspruch 1 oder 2, dadurch gekennzeichnet, daß er auch einen oder mehrere Viren von anderen IBV-Stämmen umfaßt.

4. Impfstoff nach Anspruch 3, dadurch gekennzeichnet, daß er neben dem Virus des Stammes 84-3 auch ein Virus des Stammes H120 umfaßt.

5. Impfstoff nach Anspruch 4, dadurch gekennzeichnet, daß die Gesamtmenge von IBV wenigstens $10^{6.0}$ EID$_{50}$ pro Dosis beträgt.

6. Impfstoff nach den Ansprüchen 1-5, dadurch gekennzeichnet, daß er auch ein oder mehrere andere Geflügelviren umfaßt.

7. Impfstoff nach Anspruch 6, dadurch gekennzeichnet, daß er neben Viren eines oder mehrerer IBV-Stämme auch das Newcastle Disease Virus umfaßt.

8. Verwendung von Virus-Stamm IBV 84-3, eingetragen beim Insitut Pasteur in Paris unter der Nr. I-473, für die Herstellung eines Lebendimpfstoffes, der zum Impfen von tagealte Küken gegen infektiöse Bronchitis geeignet ist.

9. Virus-Stamm IBV-84-3, der beim Institut Pasteur in Paris unter der Nr. I-473 eingetragen ist.